# EUROPEAN PATENT APPLICATION

(11) **EP 3 977 967 A1**
(43) Date of publication of application: **06.04.2022**
(21) Application number: 21200684.5
(22) Date of filing: 04.10.2021
(51) Int. Cl.: A61F 5/455, A61F 5/44

(54) **URINARY RELIEF DEVICE**

(30) Priority: 02.10.2020 US 202063087015 P; 28.09.2021 US 202117487635
(71) Applicant: B/E Aerospace, Inc., Winston-Salem, NC 27105 (US)
(72) Inventor: VANINETTI, Travis J., Bothell, WA (US); PEARSON, Matthew R., Hartford, CT (US); MCKEE, Jefferey M., Duvall, WA (US); TSAI, Hsin-I, Newport Pagnell (GB); WEEKS, Samantha, Winston-Salem, NC (US); JOHNSON, Glenn A., Rural Hall, NC (US)
(74) Representative: Dehns

(57) **Abstract**

A female urinary relief device is disclosed. The device may include an intimate interface device (IID) (102) configured to be positioned intimate with a user's body. The IID may include a tray (110) including a raised perimeter (116) forming a seal between the user's body and the tray. The IID may include a fluid cavity (114) configured to capture fluid from the user's body. The IID may include a pad (112) including a slot (120) configured to provide an unobstructed route to the fluid cavity. The device may include a fluid removal system configured to remove the fluid from the fluid cavity and route the fluid to a storage device. The fluid removal system may include a suction sub-system (124) configured to pull the fluid against gravity to remove the fluid from the fluid cavity. The fluid removal system may include discharge hoses (106) configured to fluidically and mechanically couple the IID to the storage device.

## Description

### BACKGROUND

Aircrew often need to urinate multiple times during flights without removing restraint systems and flight equipment.

### SUMMARY

A urinary relief device is disclosed in accordance with one or more embodiments of the present disclosure. The urinary relief device includes an intimate interface device configured to be positioned intimate with a portion of user's body. The intimate interface device includes a tray including a raised perimeter on a portion of an outer edge of the tray, the raised perimeter forming a seal between the portion of the user's body and the tray. The intimate interface device includes a fluid cavity defined by one or more internal surfaces of the tray, the fluid cavity configured to capture fluid from the portion of the user's body. The intimate interface device includes a pad positioned adjacent to an opening of the fluid cavity, the pad including a slot configured to provide an unobstructed route to the opening of the fluid cavity. The urinary relief device further includes a fluid removal system configured to remove the fluid from the fluid cavity of the intimate interface device and route the fluid to a storage device. The fluid removal system includes a suction sub-system configured to pull the fluid against gravity to remove the fluid from the fluid cavity. The fluid removal system includes one or more discharge hoses configured to fluidically and mechanically couple the intimate interface device to the storage device, the one or more discharge hoses including at least one inlet hose configured to fluidically couple the intimate interface device to the suction sub-system and at least one outlet hose configured to fluidically couple the suction sub-system to the storage device, the at least one inlet hose arranged substantially parallel with the intimate interface device when a user is in a seated positioned.

In some embodiments, the pad and the tray may be coupled together via an interlocking assembly including a protrusion and a groove.

In some embodiments, the pad may include a locator configured to allow for proper placement between a user's labia majora.

In some embodiments, the pad may include a protrusion configured to be inserted into a vaginal opening of a user.

In some embodiments, the pad may be formed of a wicking material to capture the fluid from the portion of the user's body.

In some embodiments, the suction sub-system may include one or more cartridges; a motive fluid port; and an air ejector configured to generate suction to pull the fluid from at least one of the pad or the fluid cavity.

In some embodiments, the suction sub-system may further include a human-machine interface (HMI) configured to activate the suction sub-system to pull the fluid away from the intimate interface device into the storage device.

In some embodiments, the intimate interface device may further include one or more sensors configured to detect the fluid to one of activate or deactivate the one or more cartridges of the suction sub-system.

In some embodiments, the air ejector may be coated with one or more anti-microbial coatings.

In some embodiments, the one or more cartridges may include one or more carbon dioxide cartridges.

In some embodiments, the fluid removal system may further include one or more leg straps configured to secure the urinary relief device to one or more portions of a user's leg.

In some embodiments, the storage device may include a quick connect port configured to receive a portion of the at least one outlet hose to fluidically and mechanically couple the suction sub-system to the storage device.

In some embodiments, the storage device may include a check valve.

In some embodiments, the storage device may include one or more gelling agents configured to increase a viscosity of the fluid.

A urinary relief device is disclosed in accordance with one or more embodiments of the present disclosure. The urinary relief device includes an intimate interface device configured to be positioned intimate with a portion of user's body. The intimate interface device includes a tray including a raised perimeter on a portion of an outer edge of the tray, the raised perimeter forming a seal between the portion of the user's body and the tray. The intimate interface device includes a fluid cavity defined by one or more internal surfaces of the tray, the fluid cavity configured to capture fluid from the portion of the user's body. The intimate interface device includes a pad positioned adjacent to an opening of the fluid cavity, the pad including a slot configured to provide an unobstructed route to the opening of the fluid cavity. The urinary relief device further includes a fluid removal system configured to remove the fluid from the fluid cavity of the intimate interface device and route the fluid to a storage device. The fluid removal system includes a suction sub-system configured to pull the fluid against gravity to remove the fluid from the fluid cavity. The suction sub-system includes one or more cartridges; a motive fluid port; and an air ejector configured to generate suction to pull the fluid from at least one of the pad or the fluid cavity. The fluid removal system includes one or more discharge hoses configured to fluidically and mechanically couple the intimate interface device to the storage device, the one or more discharge hoses including at least one inlet hose configured to fluidically couple the intimate interface device to the suction sub-system and at least one outlet hose configured to fluidically couple the suction sub-system to the storage device, the at least one inlet hose arranged substantially parallel with the intimate interface device when a user is in a seated positioned.

This Summary is provided solely as an introduction to subject matter that is fully described in the Detailed Description and Drawings. The Summary should not be considered to describe essential features nor be used to determine the scope of the Claims. Moreover, it is to be understood that both the foregoing Summary and the following Detailed Description are examples and explanatory only and are not necessarily restrictive of the subject matter claimed.

### BRIEF DESCRIPTION OF THE DRAWINGS

The detailed description is described with reference to the accompanying figures. The use of the same reference numbers in different instances in the description and the figures may indicate similar or identical items. Various embodiments or examples ("examples") of the present disclosure are disclosed in the following detailed description and the accompanying drawings. The drawings are not necessarily to scale. In general, operations of disclosed processes may be performed in an arbitrary order, unless otherwise provided in the claims. In the drawings:
FIG. 1 illustrates a perspective view of a urinary relief device, in accordance with one or more embodiments of the present disclosure.
FIG. 2A illustrates an exploded view of an intimate interface device of the urinary relief device, in accordance with one or more embodiments of the present disclosure.
FIG. 2B illustrates a front perspective view of the intimate interface device, in accordance with one or more embodiments of the present disclosure.
FIG. 2C illustrates a side view of the intimate interface device, in accordance with one or more embodiments of the present disclosure.
FIG. 2D illustrates a top view of the intimate interface device, in accordance with one or more embodiments of the present disclosure.
FIG. 3 illustrates a perspective view of a fluid removal system of the urinary relief device, in accordance with one or more embodiments of the present disclosure.
FIG. 4 illustrates a front view of a storage device of the urinary relief device, in accordance with one or more embodiments of the present disclosure.
FIG. 5A illustrates an example implementation of the urinary relief device, in accordance with one or more embodiments of the present disclosure.
FIG. 5B illustrates an example implementation of the urinary relief device, in accordance with one or more embodiments of the present disclosure.
FIG. 6 illustrates a flowchart depicting a method or process for assembling/installing the urinary relief device, in accordance with one or more embodiments of the present disclosure.
FIG. 7 illustrates a flowchart depicting a method or process for using the urinary relief device once assembled/installed, in accordance with one or more embodiments of the present disclosure.

### DETAILED DESCRIPTION

Before explaining one or more embodiments of the disclosure in detail, it is to be understood that the embodiments are not limited in their application to the details of construction and the arrangement of the components or steps or methodologies set forth in the following description or illustrated in the drawings. In the following detailed description of embodiments, numerous specific details may be set forth in order to provide a more thorough understanding of the disclosure. However, it will be apparent to one of ordinary skill in the art having the benefit of the instant disclosure that the embodiments disclosed herein may be practiced without some of these specific details. In other instances, well-known features may not be described in detail to avoid unnecessarily complicating the instant disclosure.

As used herein a letter following a reference numeral is intended to reference an embodiment of the feature or element that may be similar, but not necessarily identical, to a previously described element or feature bearing the same reference numeral (e.g., 1, 1a, 1b). Such shorthand notations are used for purposes of convenience only and should not be construed to limit the disclosure in any way unless expressly stated to the contrary.

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

In addition, use of "a" or "an" may be employed to describe elements and components of embodiments disclosed herein. This is done merely for convenience and "a" and "an" are intended to include "one" or "at least one," and the singular also includes the plural unless it is obvious that it is meant otherwise.

Finally, as used herein any reference to "one embodiment" or "some embodiments" means that a particular element, feature, structure, or characteristic described in connection with the embodiment is included in at least one embodiment disclosed herein. The appearances of the phrase "in some embodiments" in various places in the specification are not necessarily all referring to the same embodiment, and embodiments may include one or more of the features expressly described or inherently present herein, or any combination of or sub-combination of two or more such features, along with any other features which may not necessarily be expressly described or inherently present in the instant disclosure.

Aircrew often need to urinate multiple times during flights without removing restraint systems and flight equipment. In particular, female aviators (such as Air Force pilots) often need to relieve themselves during flight without interfering with operations or compromising flight safety. The current mission profiles and air refueling capabilities (up to 16 hours) have led to longer flight times for aircrew. Conventional urinary devices are bulky, uncomfortable, difficult to use, and not leak proof. Further, often times female aviators are seated in a seated position where the seat has a slight incline. Conventional urinary devices often use gravity to discharge the fluid. Therefore, there is a need to provide a urinary device aimed to address the unmet bladder relief needs of females, specifically female aircrew members. In particular, there is a need to provide an on-demand, ergonomic, discreet, and sterile device aimed to address the unmet bladder relief needs of females (e.g., female aircrew members). Further, there is a need for a device that pulls fluid against gravity when discharging the fluid such that the fluid is not contained in the fluid cavity for an extended period of time.

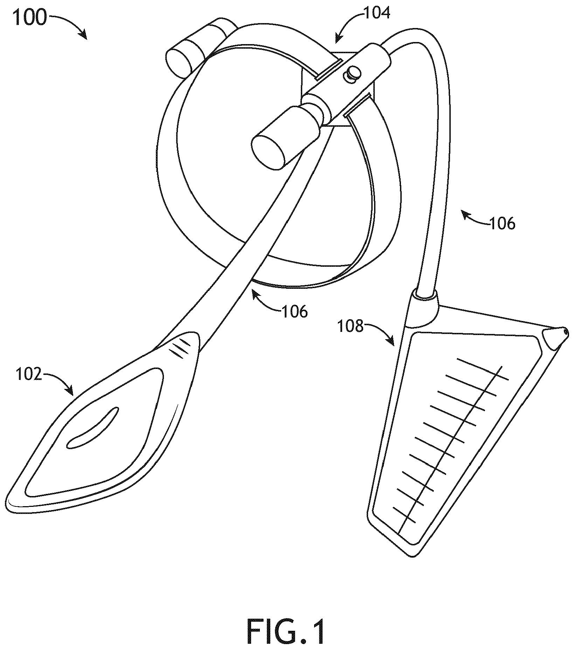

FIG. 1 illustrates a perspective view of a urinary relief device 100, in accordance with one or more embodiments of the present disclosure.

The device 100 may include an intimate interface device 102. The intimate interface device 102 may be configured to be placed intimate with a portion of a user's body. For example, the intimate interface device 102 may be configured to be held in place between the body and a user's undergarments (e.g., underwear) and/or garments. For instance, a user (e.g., an aircrew member) may quickly and discretely place the intimate interface device 102 between their body and underwear (or other garments) prior to use (e.g., pre-flight or after mission briefing). In this regard, the user may discretely wear the intimate interface device 102 prior to use and not be required to fully undress while in the cockpit (or other public area).

The intimate interface device 102 may be shaped and/or dimensioned to be placed intimately with a user's body. For example, the intimate interface device 102 may be slim such that the intimate interface device 102 may be worn discretely and comfortably for an extended amount of time (e.g., during long missions/flights). Further, the intimate interface device 102 may shaped to naturally complement the shape of the user's body. For example, the intimate interface device 102 may be asymmetrically curved to naturally complement the shape of the user's body.

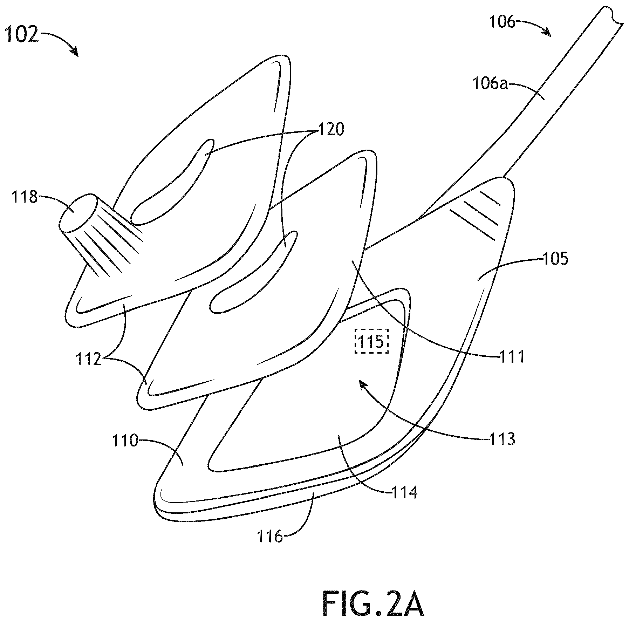

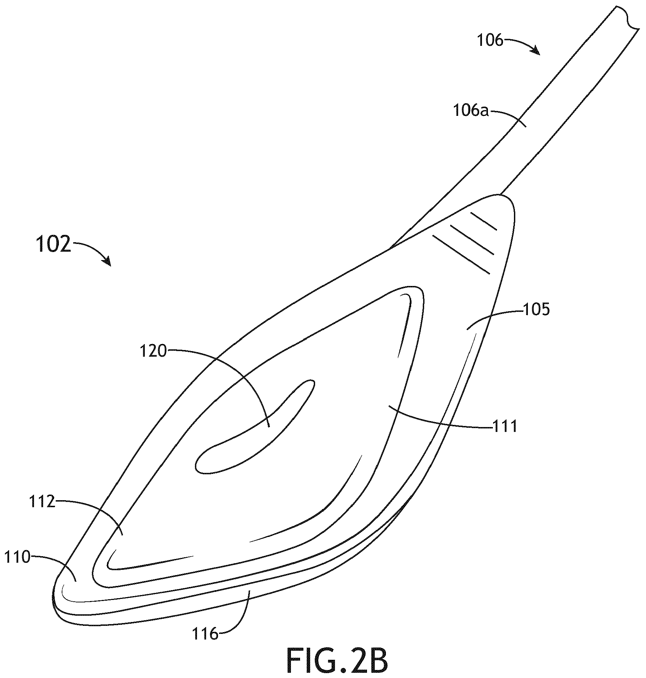

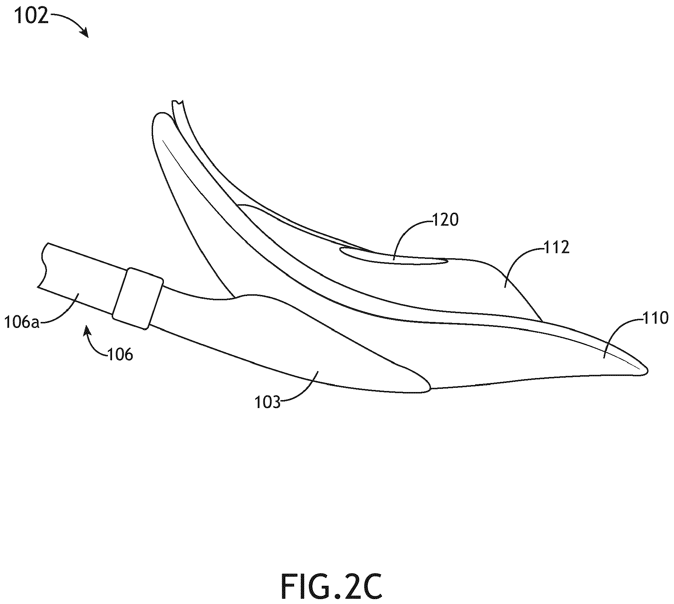

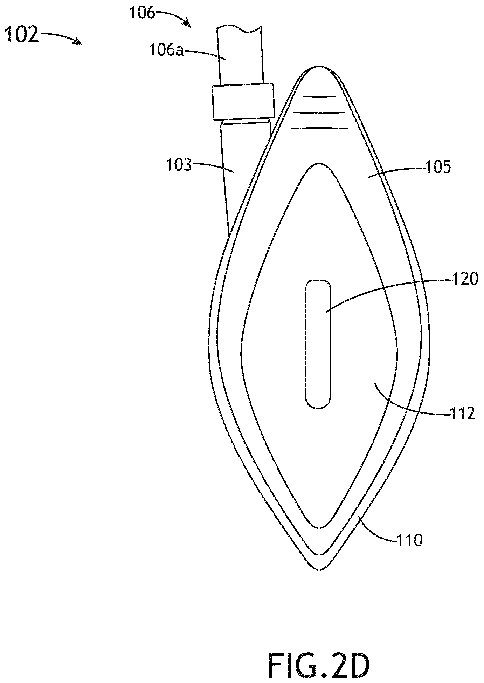

FIGS. 2A-2D illustrate various views of the intimate interface device 102, in accordance with one or more embodiments of the present disclosure. In particular, FIG. 2A illustrates an exploded view of the intimate interface device 102, in accordance with one or more embodiments of the present disclosure. In particular, FIG. 2B illustrates a front perspective view of the intimate interface device 102, in accordance with one or more embodiments of the present disclosure. In particular, FIG. 2C illustrates a side view of the intimate interface device 102, in accordance with one or more embodiments of the present disclosure. In particular, FIG. 2D illustrates a top view of the intimate interface device 102, in accordance with one or more embodiments of the present disclosure.

Referring to FIG. 2A, the intimate interface device 102 may include, but is it not limited to, a tray 110, a pad 112, and a fluid cavity 114.

In some embodiments, the tray 110 may be a reusable tray 110, such that the tray 110 may be detached after each use (e.g., mission). In such example, the tray 110 may be detached after each use (e.g., mission) and washed using a cleaning agent or detergent (or other cleaning solution), such that the previously washed tray 110 may be reused in a further mission. In alternative/additional embodiments, the tray 110 may be a single use tray 110, such that the tray 110 may be disposed of after each use and a new single use tray 110 may be attached prior to each use.

The tray 110 may include a raised perimeter 116 on an outer edge of the tray 110. For example, the raised perimeter 116 may be configured to create a seal between the body and the tray 110 of the intimate interface device 102. In this regard, the seal formed between the raised perimeter 116 and the body may be configured to prevent leakage during use. It is noted that the tray 110 may be formed of any flexible material configured to flex with the body during use, such that the intimate interface device 102 may be worn comfortably for an extended period of time. For example the tray 110 may be formed of medical grade silicone.

The fluid cavity 114 may be defined by one or more surfaces of the tray 110. The fluid cavity 114 may be configured to capture fluid from the user's body. For example, the fluid cavity 114 may be configured to capture urine from the user's urethra opening. By way of another example, the fluid cavity 114 may be configured to capture menstrual blood from the user's vaginal opening.

The pad 112 may be configured to reversibly, mechanically couple to at least a portion of a surface of the tray 110. For example, the pad 112 may be configured to cover an opening 113 in the fluid cavity. It is noted herein that the pad 112 may couple to the tray 110 via any coupling or fastening mechanism known in the art including, but not limited to, a press-fit assembly, one or more adhesives, one or more hook and loop mechanisms, and the like.

The pad 112 may include a slot 120 configured to provide an unobstructed route to the fluid cavity 114. For example, the fluid cavity 114 may be configured to collect the fluid from the slot 120 of the pad 112. For instance, the fluid from the pad 112 may be routed through the slot 120 to the fluid cavity 114.

The pad 112 may include a pull tab configured to allow for easy interchangeability. In this regard, the pad 112 may be a disposable pad 112 (e.g., the pad 112 is disposed of after each use), such that a new pad 112 may be re-used. In some embodiments, the pad 112 may be a reusable pad 112 (e.g., a washable pad 112), such that the reusable pad 112 may be cleaned after each use and then re-used again. The pad 112 may be formed of any wicking material known in the art suitable for directing fluid (e.g., urine) into the tray 110 and keeping the skin dry. For example, the pad 112 may be formed of a cotton mixture, such as cotton flannel. By way of another example, the pad 112 may be formed of hemp. By way of another example, the pad 112 may be formed of bamboo. It is noted herein that the pad 112 may be a variety of sizes (e.g., small, medium, large, extra-large, and the like) to accommodate different anatomies.

Referring to FIG. 2D, the pad 112 may be shaped such that the outer ridges 111 of the pad 112 may be configured to sit between the labia majora of the user. For example, the pad 112 may include a locator 122 configured to allow for proper placement during use (e.g., flight). In this regard, the wicking material of the pad 112 may be configured to be positioned as close to the urethra as possible, such that spray is mitigated.

In some embodiments, the pad 112 is configured to support menstruation. For example, the pad 112 may include a protrusion 118 configured to be inserted into the vaginal opening of a user (e.g., an aircrew member). In this regard, the protrusion 118 may be configured to allow for proper placement of the pad 112, such that during menstruation, menstrual blood does not leak during use (e.g., flight). It is noted that the protrusion 118 of the pad 112 may eliminate the need for the user to wear menstrual products (e.g., tampons, menstrual cups, menstrual napkins/pads) during flight and eliminate the need for the user to change such menstrual products during flight (e.g., tampons, menstrual cups, menstrual napkins/pads), which may decrease the risk of a toxic shock syndrome (TSS).

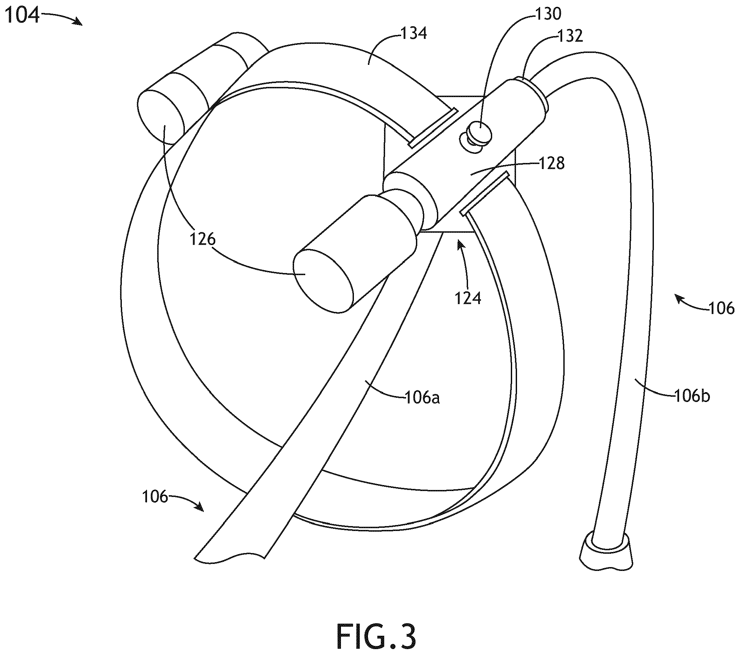

FIG. 3 illustrates a perspective view of the fluid removal system 104, in accordance with one or more embodiments of the present disclosure.

The fluid removal system 104 may be configured to pull away fluid (e.g., urine, menstrual blood, or the like) from the intimate interface device 102 as it is expressed (e.g., while the aircrew member relieves their bladder). It is noted herein that the fluid removal system 104 may be configured to provide on-demand bladder relief for users (e.g., aircrew members).

The fluid removal system 104 may include one or more discharge hoses 106 configured to fluidically and mechanically couple the intimate interface device to a storage device 108. For example, the intimate interface device 102 may reversibly, mechanically couple to a portion of the fluid removal system 104 via one or more discharge hoses 106. The one or more discharge hoses 106 (or discharge tubes) may include at least one inlet hose 106a configured to fluidically and mechanically couple the intimate interface device 102 to the fluid removal system 104. For example, as shown in FIG. 2C, the tray 110 may include a connection portion 103 configured to couple to a portion of the inlet hose 106a. The connection portion 103 may be positioned near the fluid cavity 114 of the intimate interface device 102. In this regard, the fluid (e.g., urine, menstrual blood, or the like) from the intimate interface device 102 may be directed from the intimate interface device 102 (e.g., the pad 112) to the fluid removal system 104 through the inlet hose 106a. It is noted that the inlet hose 106a may be configured to couple to the intimate interface device 102 (e.g., the tray 110) via any connection mechanism known in the art including, but not limited to, interference fitting mechanisms, slot-and-groove mechanism, or the like.

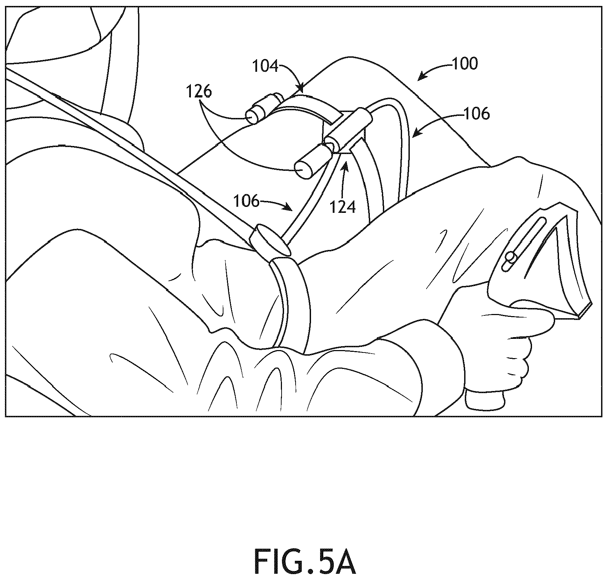

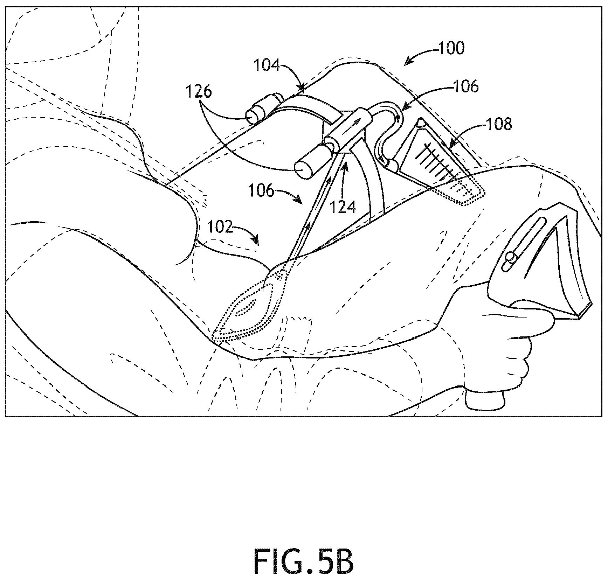

The at least one inlet hose 106a may be arranged substantially parallel with the intimate interface device when the user in the seated position (as shown in FIGS. 5A-5B). For example, when the at least one inlet hose 106a is coupled to the connection portion 103, the at least one inlet hose 106a may be positioned between the user's legs. In this regard, the inlet hose 106a may be routed through an opening in the user's garments during use.

The one or more discharge hoses 106 (or discharge tubes) may include at least one outlet hose 106b configured to fluidically and mechanically couple the fluid removal system 104 to the storage device 108. In this regard, the fluid from the fluid removal system 104 may be discharged into the storage device 108 through the outlet hose 106b.

Referring to FIGS. 5A-5B, the user may be in a seated position while the device 100 is in use and the seat may have a slight incline (e.g., the intimate interface device 102 is positioned a select distance below the fluid removal system 104. The fluid removal system 104 may include a suction sub-system 124 configured to suction or pump the fluid (e.g., urine) to remove the fluid from the fluid cavity 114 of the intimate interface device 102 to the storage device 108. For example, as shown in FIGS. 5A-5B, the suction sub-system 124 may be configured to pump the fluid against gravity from the intimate interface device 102 and route the fluid to the fluid removal system 104.

The suction sub-system 124 may include one or more cartridges 126. The one or more cartridges 126 may any type of cartridge suitable for acting as a motive fluid. For example, the one or more cartridges 126 may include one or more carbon dioxide cartridges 126. By way of another example, the one or more cartridges 126 may include one or more compressed gaseous cartridges 126 filled with any type of compressed gas. By way of another example, the one or more cartridges 126 may include one or more compressed air cartridges 126.

The one or more cartridges 126 may support any flow rate suitable for directing the fluid (e.g., urine) from the intimate interface device 102 to the storage device 108. For example, the one or more cartridges 126 may be configured to support a flow rate up to 45 ml/s.

In some embodiments, at least one of the one or more cartridges 126 may be a back-up cartridge. For example, if carbon dioxide or other compressed gaseous/air of a first cartridge is depleted, carbon dioxide or other compressed gaseous/air within an additional cartridge may be used. In this regard, the carbon dioxide or other compressed gaseous/air supply of the urinary relief device 100 may not be exhausted during a longer mission (e.g., a 16+ hour mission). It is noted herein that the one or more cartridges 126 may be disposable, such that the cartridges may be replaced once the carbon dioxide or other gaseous/air supply level of the cartridges is below a certain threshold.

The suction sub-system 124 may further include a motive fluid port 128 and an air ejector 132. For example, the one or more cartridges 126 may be coupled to the air ejector 132 via the motive fluid port 128.

The air ejector 132 may be configured to generate suction to pull fluid (e.g., urine) from the intimate interface device 102 to the storage device 108. An interior surface of the air ejector 132 may be coated with one or more anti-microbial coatings. In this regard, the anti-microbial coatings may be configured to prevent microbe build-up or growth inside the air ejector 132. Thus, preventing possible bacterial infections in aircrew members who use the urinary relief device 100 during flight. It is noted herein that after use, the air ejector 132 and the outlet hose 106b may be rinsed with soapy water and then dried. Alternatively, soapy water may be run through the urinary relief device 100.

In some embodiments, the suction sub-system 124 may include one or more human-machine interfaces 130 (HMI). For example, as shown in FIG. 3, the one or more HMI 130 may be coupled to a portion of the motive fluid port 128. The HMI 130 may be configured to start the flow of carbon dioxide contained within the one or more carbon dioxide cartridges 126 to begin void. For example, the HMI 130 may include a button. For instance, when the button is pressed, the flow of carbon dioxide contained within the one or more carbon dioxide cartridges 126 begins to flow and void begins. It is noted that the HMI 130 may include any type of HMI known in the art such as, but not limited to, a button, a toggle, or the like.

In alternative/additional embodiments, the suction sub-system 124 may include one or more sensors 115. For example, the tray 110 of the intimate interface device 102 may include one or more sensors 115 configured to detect when fluid is present and activate/deactivate the air cartridge pump automatically in response to the detection result. In one instance, the one or more sensors 115 may be a standalone component of the intimate interface device 102 and be coupled to one or more surfaces of the fluid cavity 114 to detect when fluid is present. In another instance, the one or more sensors 115 may be integral to the tray 110 of the intimate interface device (e.g., by depositing metallic electrodes onto the electrically insulating material that forms the tray 110, or the like). In this regard, the one or more sensors 115 may allow for hands-free operation of the urinary relief device 100. It is noted herein that the one or more sensors 115 may be in lieu of or in addition to the HMI 130, therefore the above discussion shall not be construed as a limitation on the scope of the present disclosure.

The one or more sensors 115 may be powered by a power source (e.g., battery) housed in one or more components of the urinary device 100. For example, the power source may be housed in the fluid removal system 104. In this instance, a pair of connection lines (e.g., wires) may carry the power from the fluid removal system 104 to the sensors 115. By way of another example, the power source may be housed in the intimate interface device 102. In this instance, a pair of connection lines (e.g., wires) may carry the signal from the sensors to the fluid removal system 104..

It is noted that the one or more sensors 115 may include any type of sensor suitable for detecting when fluid is present. For example, the one or more sensors 115 may include one or more electrical liquid sensors including, but not limited to, one or more optical sensors, one or more capacitive sensors, one or more conductive electrical sensors, or the like. It is noted that the one or more sensors 115 may be arranged on any location of the tray 110. Further, the tray 110 may include any number of sensors suitable for detecting when fluid is present. FIG. 2A is provided merely for illustrative purposes and shall not be construed as limiting the scope of the present disclosure.

The fluid removal system 104 may include a leg strap 134 configured to secure the urinary relief device 100 to a portion of a user's leg (e.g., an aircrew member's leg). For example, the leg strap 134 may be configured to secure the urinary relief device 100 to a portion of a user's left leg (e.g., an aircrew member's left leg). By of another example, the leg strap 134 may be configured to secure the urinary relief device 100 to a portion of an aircrew member's right leg. The leg strap 134 may enable optimal user placement, such that the urinary relief device 100 may be positioned comfortably. In this regard, the fluid removal system 104 may be slipped onto a user's leg via the leg strap 134 while on-board and the inlet hose 106a may be connected to the intimate interface device 102, then the inlet hose 106a may be routed through a zipper in the member's garments (e.g., flight suit) to the fluid removal system 104.The fluid removal system 104 may be activated and voided, such that the fluid removal system 104 may pull fluid away from the intimate interface device 102 into the storage device 108.

The leg strap 134 may be configured to support users of various sizes. For example, the leg strap 134 may be adjustable, such that the circumference of the leg strap 134 may be adjusted to support varying leg circumferences.

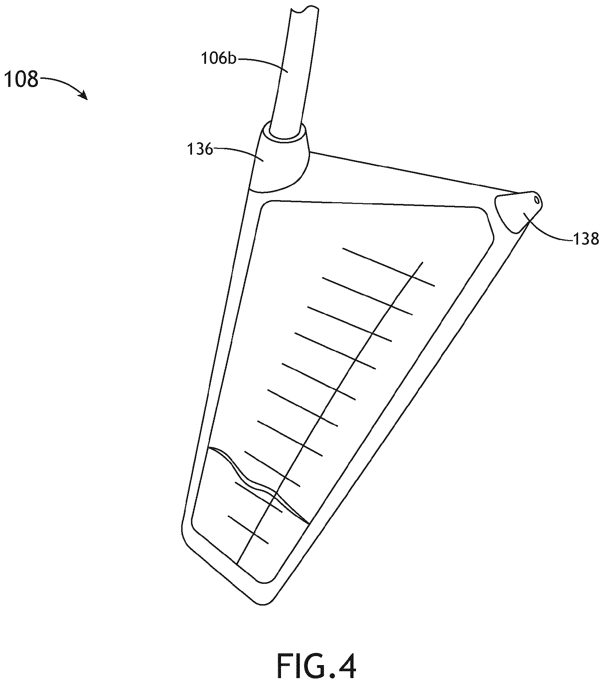

FIG. 4 illustrates a front view of the storage device 108, in accordance with one or more embodiments of the present disclosure.

The storage device 108 may include a quick connect port 136 configured to fluidically and mechanically couple the fluid removal system 104 to the storage device 108. For example, the quick connect port 136 may be configured to mechanically couple to the outlet hose 106b. In this regard, the outlet hose 106b fluidically couples the fluid removal system 104 to the storage device 108, such that fluid (e.g., urine) from the fluid removal system 104 may be directed to the storage device 108.

The storage device 108 may be secured to the aircrew member's leg during flight by attaching the storage device 108 with a leg band. For example, the leg strap 134 may be configured to secure the storage device 108 to the member's leg during flight. By way of another example, an additional leg strap or band may be used to secure the storage device 108 during flight. In this regard, the storage device 108 is secured such that the contents of the storage device 108 (e.g., urine) does not spill or leak during flight.

It is noted herein that after use, the storage device 108 may be detached from the outlet hose 106b via the quick connect port 136. In this regard, the storage device 108 may be disposed of at the end of the mission.

The storage device 108 may include a check valve 138. The check valve 138 may be configured to reduce odors associated with the contents of the storage device 108 (e.g., reduce any odors associated with urine).

The storage device 108 may further include one or more gelling agents and deodorizers. For example, the one or more gelling agents may be configured to increase the viscosity of the fluid (e.g., urine), such that the fluid (e.g., urine) is in a gel-like form (rather than a liquid form). By way of another example, the one or more deodorizers may be configured to reduce any odors associated with the urine or other contents within the storage device 108.

The storage device 108 may include any storage device 108 known in the art. For example, the storage device 108 may include one or more storage bags 108. For instance, the storage device 108 may include one or more disposable medical-grade PVC storage bags.

The storage device 108 may be configured to support up to 4 liters (4L) of fluid storage. For example, the storage device 108 may be an expanding storage device 108 configured to support up to 4L of fluid. In this regard, the urinary relief device 100 may be used during a longer mission (e.g., a 16+ hour mission). The urinary relief device 100 may be configured to support various sized storage devices 108 such that the device 100 may be used during various missions and for optimal positioning/use.

The storage device 108 may be translucent (or partially translucent) such that the aircrew member may see the contents of the storage device 108. The storage device may further include one or more graduation marks such that the aircrew member may be able to quantify an amount of urine within the storage device 108.

The one or more discharge hoses 106 (e.g., the inlet hose 106a and the outlet hose 106b) may be formed of any material known in the art. For example, the one or more connection mechanisms 106 may be formed of PVC tubing and covered with a protective sleeve. In this regard, during flight, the one or more connection mechanisms 106 are protected from one or more external objects.

It is noted herein that one or more components of the urinary relief device 100 may be configured to provide comfort for all-day wearability and prevent leakage during bladder relief. For example, the intimate interface device 102 may be asymmetrically shaped/ergonomically shaped such that an aircrew member (e.g., a pilot) may route the connection mechanism 106 (e.g., the outlet hose) around underwear and out through the flight suit zipper.

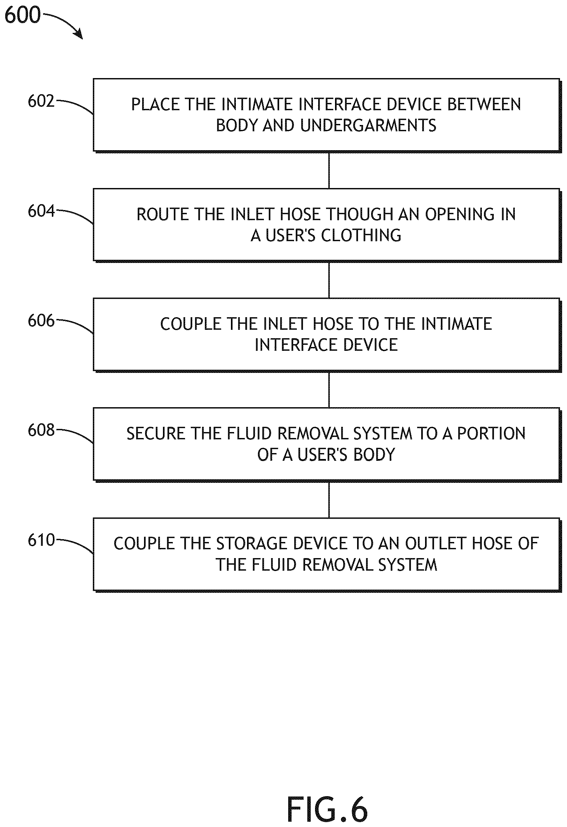

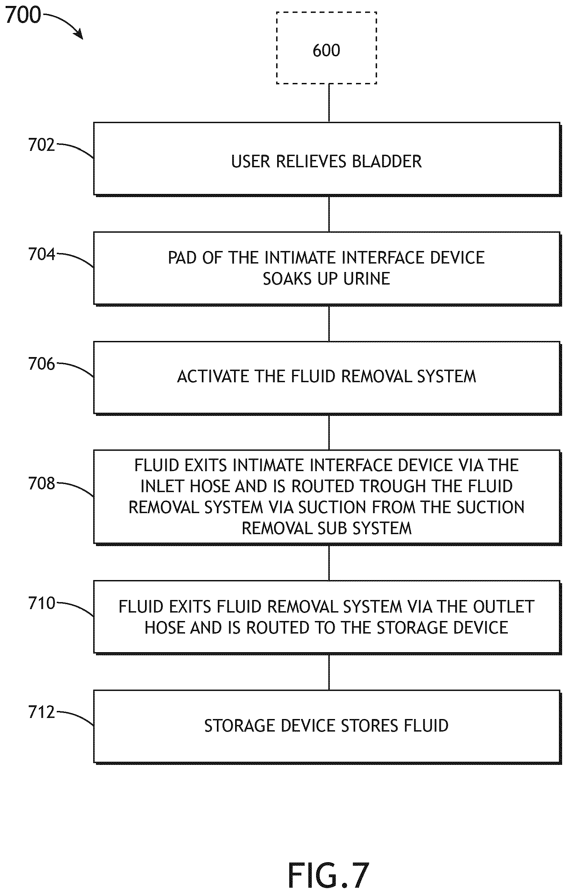

FIGS. 5A-5B illustrate a user wearing the urinary relief device 100, in accordance with one or more embodiments of the present disclosure. FIG. 6 illustrates a flowchart depicting a method or process 600 for assembling/installing the urinary relief device 100, in accordance with one or more embodiments of the present disclosure. FIG. 7 illustrates a flowchart depicting a method or process 700 for using the urinary relief device 100 once the device 100 is installed/assembled (using method or process 600), in accordance with one or more embodiments of the present disclosure.

In a step 602, the intimate interface device 102 may be placed intimately with a portion of a user's body. For example, the intimate interface device 102 may be placed between a user's body and a user's undergarments (as shown in FIGS. 5A-5B).

In a step 604, the inlet hose 106a may be routed through an opening in the user's clothing. In some embodiments, prior to routing the inlet hose 106a through an opening in the user's clothes (e.g., through a zipper opening), the fluid removal system 104 may be secured to a portion of the user's body (discussed below with respect to step 608).

In a step 606, the intimate interface device 102 may be coupled to the fluid removal system 104. For example, the inlet hose 106a of the fluid removal system 104 may be coupled to the connection portion 103 of the intimate interface device 102.

In a step 608, the fluid removal system 104 may be secured to a portion of a user's body. For example, the leg strap 134 of the fluid removal system 104 may be secured to a portion of a user's leg.

In a step 610, the storage device 108 may be coupled to the outlet hose 106b of the fluid removal system 104.

Once the urinary relief device 100 has been assembled/installed (steps 602-610), the urinary device 100 may be ready for use during flight.

In a step 702, a user relieves their bladder during flight.

In a step 704, the pad 112 of the intimate interface device 102 may capture (or wick) fluid (e.g., urine) into the intimate interface device 102. For example, the pad 112 may be formed of a wicking material suitable for capturing/wicking additional moisture as need.

In a step 706, the fluid removal system 104 may remove the liquid from the pad 112 upon activation. For example, a user may activate the fluid removal system 104 (or deactivate) via the HMI 130. For instance, a user may press the button 130 to start flow of carbon dioxide (or other compressed air/gas) and begin void. In this regard, the suction sub-system 124 may be configured to suction/pump the liquid from the pad 112 upon activation via the HMI 130 (e.g., pressing the button 130). By way of another example, the one or more sensors 115 may be configured to activate the fluid removal system 104 (or deactivate the fluid removal system 104) upon detection of fluid within a portion of the intimate interface device.

In a step 706, the fluid may exit the intimate interface device 102 via the inlet hose 106a and be routed through the fluid removal system 104. For example, the urine may be routed to the inlet hose 106a and through the motive fluid port 128. For instance, suction may be generated from the ejector 132 and urine may be pulled from the intimate interface device 102 through the fluid removal system 104. In this regard, the urine may be pulled against gravity and routed to the remote storage device 108.

In a step 708, the fluid may exit the fluid removal system 104 via the outlet hose 106b and be routed to the storage device 108. For example, the urine may be ejected from the fluid removal system 104 and routed to the outlet hose 106b. The outlet hose 106b may be fluidically coupled to the storage device 108, such that the fluid flows into the storage device 108.

In a step 710, the storage device may store the fluid. For example, the storage device 108 coupled to the outlet hose 106b may receive the urine and store the urine until discharge. In some embodiments, the storage device 108 may include a gelling agent configured to convert the liquid urine into a gel.

Although embodiments of the disclosure are directed to an aviation environment, it is noted herein the urinary relief device 100 is not limited to use in an aviation environment. For example, the urinary relief device 100 may be used in conjunction with autonomous drone vehicle environments. Therefore, the above description should not be interpreted as a limitation on the present disclosure but merely an illustration.

It is to be understood that embodiments of the methods disclosed herein may include one or more of the steps described herein. Further, such steps may be carried out in any desired order and two or more of the steps may be carried out simultaneously with one another. Two or more of the steps disclosed herein may be combined in a single step, and in some embodiments, one or more of the steps may be carried out as two or more sub-steps. Further, other steps or sub-steps may be carried in addition to, or as substitutes to one or more of the steps disclosed herein.

Although inventive concepts have been described with reference to the embodiments illustrated in the attached drawing figures, substitutions made herein without departing from the scope of the claims. Components illustrated and described herein are merely examples of a system/device and components that may be used to implement embodiments of the inventive concepts and may be replaced with other devices and components without departing from the scope of the claims. Furthermore, any dimensions, degrees, and/or numerical ranges provided herein are to be understood as non-limiting examples unless otherwise specified in the claims.

## Claims

1. A urinary relief device, comprising:
an intimate interface device (102) configured to be positioned intimate with a portion of a user's body, the intimate interface device comprising:
a tray (110) including a raised perimeter (116) on a portion of an outer edge of the tray, the raised perimeter forming a seal between the portion of the user's body and the tray;
a fluid cavity (114) defined by one or more internal surfaces of the tray, the fluid cavity configured to capture fluid from the portion of the user's body; and
a pad (112) positioned adjacent to an opening of the fluid cavity, the pad including a slot (120) configured to provide an unobstructed route to the opening of the fluid cavity;
a fluid removal system (104) configured to remove the fluid from the fluid cavity of the intimate interface device and route the fluid to a storage device (108), the fluid removal system comprising:
a suction sub-system (124) configured to pull the fluid against gravity to remove the fluid from the fluid cavity; and
one or more discharge hoses (108) configured to fluidically and mechanically couple the intimate interface device to the storage device,
the one or more discharge hoses including at least one inlet hose (106a) configured to fluidically couple the intimate interface device to the suction sub-system and at least one outlet hose (106b) configured to fluidically couple the suction sub-system to the storage device, the at least one inlet hose arranged substantially parallel with the intimate interface device when a user is in a seated positioned.

2. The urinary relief device of Claim 1, wherein the pad and the tray are coupled together via a press-fit assembly.

3. The urinary relief device of Claim 1 or 2, wherein the pad includes a locator configured to allow for proper placement between a user's labia majora.

4. The urinary relief device of any preceding Claim, wherein the pad includes a protrusion configured to be inserted into a vaginal opening of a user.

5. The urinary relief device of any preceding Claim, wherein the pad is formed of a wicking material configured to capture the fluid from the portion of the user's body.

6. The urinary relief device of any preceding Claim, wherein the suction sub-system comprises:
one or more cartridges (126);
a motive fluid port (128); and
an air ejector (132) configured to generate suction to pull the fluid from at least one of the pad or the fluid cavity.

7. The urinary relief device of Claim 6, wherein the suction sub-system further comprises:
a human-machine interface, HMI, (130) configured to activate the suction sub-system to pull the fluid away from the intimate interface device into the storage device.

8. The urinary relief device of Claim 6 or 7, wherein the intimate interface device further comprises:
one or more sensors (115) configured to detect the fluid to one of activate or deactivate the one or more cartridges of the suction sub-system.

9. The urinary relief device of Claim 6, 7 or 8, wherein the air ejector (132) is coated with one or more anti-microbial coatings.

10. The urinary relief device of Claim 6, 7, 8 or 9, wherein the one or more cartridges include one or more carbon dioxide cartridges.

11. The urinary relief device of any preceding Claim, wherein the fluid removal system further comprises:
one or more leg straps (134) configured to secure the urinary relief device to one or more portions of a user's leg.

12. The urinary relief device of any preceding Claim, wherein the storage device includes a quick connect port configured to receive a portion of the at least one outlet hose to fluidically and mechanically couple the suction sub-system to the storage device.

13. The urinary relief device of any preceding Claim, wherein the storage device includes a check valve.

14. The urinary relief device of any preceding Claim, wherein the storage device includes one or more gelling agents configured to increase a viscosity of the fluid.

15. A urinary relief device, comprising:
an intimate interface device (102) configured to be positioned intimate with a portion of a user's body, the intimate interface device comprising:
a tray (110) including a raised perimeter (116) on a portion of an outer edge of the tray, the raised perimeter forming a seal between the portion of the user's body and the tray;
a fluid cavity (114) defined by one or more internal surfaces of the tray, the fluid cavity configured to capture fluid from the portion of the user's body; and
a pad (112) positioned adjacent to an opening of the fluid cavity, the pad including a slot (120) configured to provide an unobstructed route to the opening of the fluid cavity;
a fluid removal system (104) configured to remove the fluid from the fluid cavity of the intimate interface device and route the fluid to a storage device (108), the fluid removal system comprising:
a suction sub-system (124) configured to pull the fluid against gravity to remove the fluid from the fluid cavity; and
one or more discharge hoses (108) configured to fluidically and mechanically couple the intimate interface device to the storage device,
the one or more discharge hoses including at least one inlet hose (106a) configured to fluidically couple the intimate interface device to the suction sub-system and at least one outlet hose (106b) configured to fluidically couple the suction sub-system to the storage device, the at least one inlet hose arranged substantially parallel with the intimate interface device when a user is in a seated positioned, wherein the suction sub-system comprises:
one or more cartridges (126);
a motive fluid port (128); and
an air ejector (132) configured to generate suction to pull the fluid from at least one of the pad or the fluid cavity.
